# EUROPEAN PATENT SPECIFICATION

(11) **EP 2 916 900 B1**
(45) Date of publication and mention of the grant of the patent: **06.11.2019**
(21) Application number: 13716500.7
(22) Date of filing: 13.03.2013
(51) Int. Cl.: A61M 25/01, B65B 5/04, B65B 7/02, A61M 25/00

(54) **VAPOR HYDRATION OF MEDICAL DEVICE WITHIN PACKAGE**
DAMPFHYDRATION EINES HYDROPHILEN KATHETERS IN EINER VERPACKUNG
HYDRATATION PAR VAPEUR D'UN CATHÉTER HYDROPHILE DANS UN EMBALLAGE

(30) Priority: 12.11.2012 US 201261725294 P
(43) Date of publication of application: 16.09.2015
(73) Proprietor: Hollister Incorporated, Libertyville, IL 60048 (US)
(72) Inventor: SZOSTAK, Robert, A., Lake Forest, IL 60045 (US); GREYNOLDS, Robert, A., Northbrook, IL 60062 (US)
(74) Representative: FRKelly
(86) International application number: PCT/US2013/030870
(87) International publication number: WO 2014/074142

(56) References cited:
- WO-A1-97/47349
- WO-A1-2004/066876
- WO-A2-2005/014055

## Description

### Related Application

This application claims the benefit of and priority to U.S. Provisional Patent Application Serial No. 61/725,294, filed November 12, 2012.

### Field of the Disclosure

This disclosure relates generally to packaging for medical devices that require hydration, activation, or wetting prior to use, such as hydrophilic medical devices, and more specifically, to packaging that achieves hydration or wetting of hydrophilic urinary catheters.

### Background of Disclosure

Intermittent catheterization is a good option for many users who suffer from various abnormalities of the urinary system. A common situation is where single use, individually packaged, sterile catheters are used. It is quite common for catheters to include a surface treatment that reduces friction to allow for easier and less traumatic insertion into and through the user's urethra. One such surface treatment includes providing a hydrophilic coating on the exterior surface of the catheter.

In a hydrophilic coated catheter, the catheter is provided with a thin hydrophilic coating which is disposed on the outer surface of the catheter. When this coating is activated by contact with a hydrating medium, such as liquid water or water vapor, it becomes lubricious and provides an extremely low coefficient of friction surface. Previously, the most common form of this product is where a sterile, individually packaged single use catheter is provided in a dry state or condition. The user opens the package, pours water into the package, waits 30 seconds, and then removes the catheter from the package, now activated and ready for insertion.

In another version of a hydrophilic coated catheter, the catheter is provided in a package that already contains enough loose liquid water to cause the catheter to be immersed within the water. For this product, the user simply opens the package and removes the catheter which is ready for insertion without the need to add water and wait 30 seconds. One disadvantage of this type of hydrophilic coated catheters is that the immersion liquid has a tendency to spill from the package as the user handles the catheter and tries to remove it for subsequent insertion.

In a more recent product, the hydrophilic catheter is vapor hydrated with a vapor medium, such as water vapor, within the catheter package. In this product, the package includes a cavity containing the hydrophilic catheter. Liquid water is sequestered within the catheter package and releases water vapor into the cavity to activate the hydrophilic catheter. WO 2005/014055 discloses a vapor hydrated hydrophilic catheter assembly. WO97/47349 discloses a multipack catheter that includes a sachet of silica gel and a sachet of active carbon. WO2004/066876 discloses packaged vascular package including two moisture absorbing packs.

The present disclosure provides medical catheters with improved vapor hydration features.

### Summary of the Disclosure

There are several aspects of the present disclosure which may be embodied separately or together in the devices and systems described and claimed below. These aspects may be employed alone or in combination with other aspects of the disclosure described herein, and the description of these aspects together is not intended to preclude the use of these aspects separately or the claiming of such aspects separately or in different combinations as set forth in the claims appended hereto.

In one aspect, a ready-to-use packaged vapor hydrated hydrophilic medical device assembly includes a package defining a sealed cavity and a medical device made at least partially from a hydrophilic material disposed within the cavity. The assembly also includes a plurality of sachets disposed within the cavity. The sachets are at least partially made of a vapor permeable, liquid impermeable material and contain a vapor donating medium therein. Vapor donated from the vapor donating medium permeates through the vapor permeable material of the sachets and into the sealed cavity defined by the package to active the hydrophilic material of the medical device.

In another aspect a ready-to-use packaged vapor hydrated hydrophilic catheter assembly includes a gas impermeable package defining a sealed cavity and a hydrophilic coated catheter disposed within the cavity. The assembly includes a plurality of sachets which are also disposed within the cavity. The sachets are at least partially formed from a vapor permeable, liquid impermeable material and contain a vapor donating medium therein. Vapor donated from the vapor donating medium permeates through the vapor permeable material of the sachets and into the sealed cavity to active the hydrophilic coating on the catheter.

In yet a further aspect, a method of manufacturing a packaged vapor hydrated hydrophilic medical device assembly includes placing a medical device comprising a hydrophilic material into a cavity of a package. A plurality of sachets containing a vapor donating medium is also placed within the cavity of the package. The sachets are at least partially formed of a vapor permeable, liquid impermeable material. The package is then sealed.

### Summary of the Invention

In accordance with the present invention, there is provided a ready-to-use packaged vapor hydrated hydrophilic medical device assembly as defined in claim 1 and a method of manufacturing a packaged vapor hydrated hydrophilic medical device assembly as defined in claim 9.

Further advantages are achieved by the embodiments indicated by the dependent claims.

### Brief Description of the Drawing

Fig. 1 is a top plan view, partially broken away, of a vapor hydrated packaged hydrophilic medical device assembly according to the present disclosure;
Fig. 2 is a cross-sectional view of the packaged medical device assembly of Fig. 1 taken along lines 2-2;
Fig. 3 is a cross-sectional view of another embodiment a vapor hydrated packaged hydrophilic medical device assembly according to the present disclosure;
Fig. 4 is a schematic side view which illustrates one embodiment of the construction of the sachets of the present disclosure;
Fig. 5 is a front view showing the construction of the sachets shown in Fig. 4;
Fig. 6 is a schematic perspective view which illustrates another embodiment of a method of the constructing sachets of the present disclosure;
Fig. 7 is a top plan view of one embodiment of the sachets of the present disclosure; and
Fig. 8 is a top plan view of another embodiment of the sachets of the present disclosure.

### Detailed Description

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims. For example, while the specific embodiments disclosed herein are described in relation to catheter assemblies, other medical devices and assemblies that require hydration prior to use may be used. Such devices and assemblies may include for example, medical implants, contact lenses, etc.

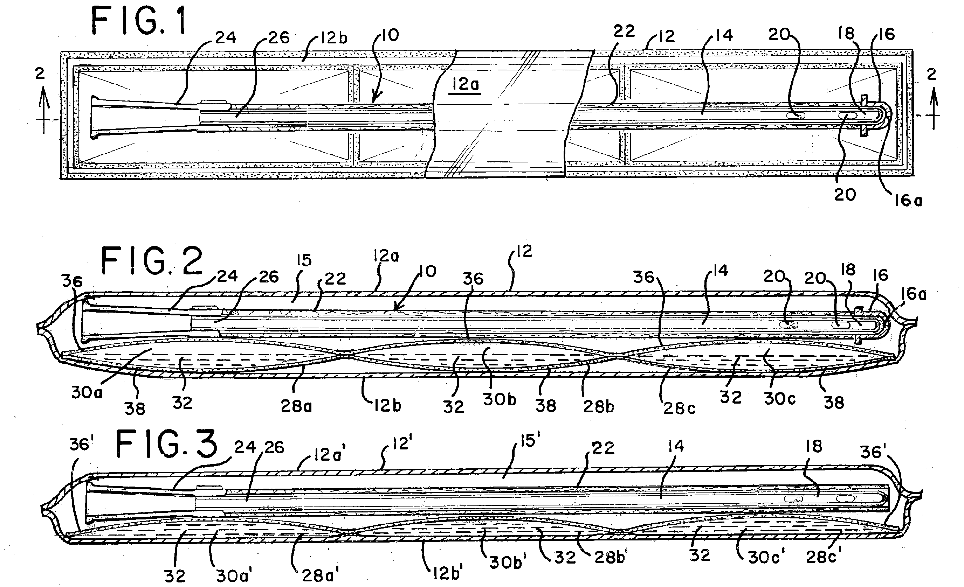

Fig. 1 illustrates a hydrophilic medical device or assembly of the present disclosure, such as the illustrated a hydrophilic catheter assembly 10, which is adapted for vapor hydration within package 12 so it is ready for immediate use when the end user opens the package 12. In other embodiments, the medical device or assembly may comprise any medical device or assembly that requires hydration prior to use. Package 12 is liquid and gas impermeable and may be formed from any suitable liquid and gas impermeable materials such as, for example, an aluminum foil or a polymer coated aluminum foil. As an alternative to aluminum foil, which is a very good water vapor barrier, other packaging materials may be chosen for other considerations, such as thermoformability or cost. As illustrated in Figs. 1 and 2, the illustrated package 12 includes a top wall 12a and a bottom wall 12b that defines a sealed cavity 15.

The catheter assembly 10 within the sealed cavity 15 of the package 12 includes a catheter tube 14 having an outer surface with a hydrophilic coating on at least a portion thereof, an optional soft, rubbery introducer tip 16 adjacent an end 18 of tube 14 intended for pre-insertion into the urethral opening before advancement of the catheter tube, and drainage eyes 20 near the proximal insertion end 18 of tube 14 for draining urine from the bladder. A connector or drainage element 24 may be located at the distal end 26 of tube 14 for connecting the catheter tube to a flexible drain tube that leads to a suitable collection container or for drainage directly to a suitable collection container. In the illustrated catheter assembly 10, the connector 24 is shown as a tapered funnel.

Also located within cavity 15 of package 12 is a plurality of individual sachets 28a, 28b and 28c, such as the illustrated packets, pouches or pillows. The sachets 28a, 28b and 28c define compartments 30a, 30b and 30c which contain a vapor donating medium or liquid 32, such as liquid water. At least a portion of sachets 28a, 28b and 28c is formed from a "breathable" (vapor permeable, but liquid impermeable) material having a high moisture vapor transmission rate (MVTR), such as, for example, polyolefin-calcium carbonate microporous film or GORE® Medical Membrane material (an expanded polytetrafluoroethylene (ePTFE) membrane). Hydrating vapor, such as water vapor, donated from the vapor donating medium 32 permeates through the vapor permeable material of the sachets 28a - 28c and into cavity 15 wherein the vapor activates or hydrates the hydrophilic coating on the catheter.

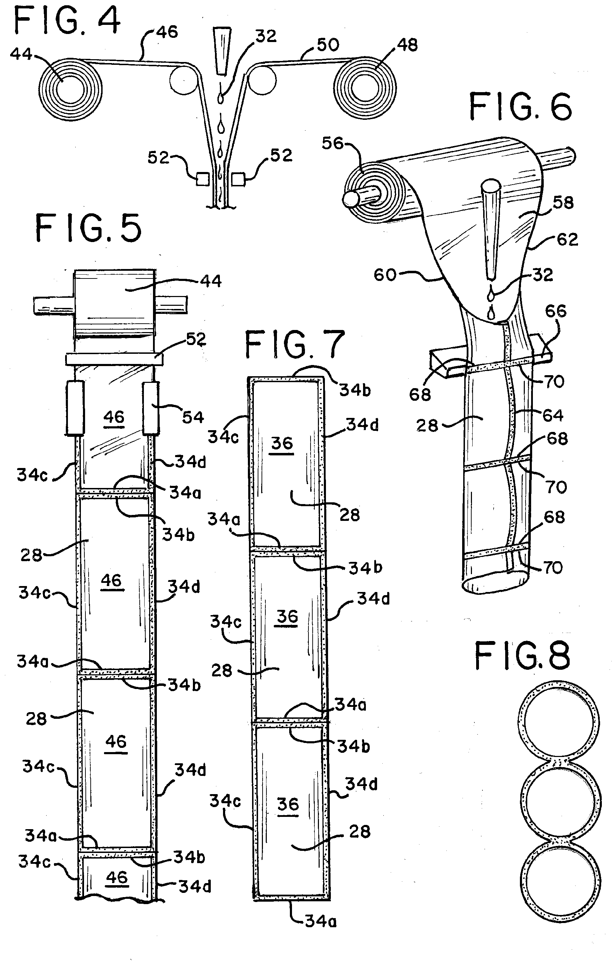

Sachets 28a, 28b and 28c may each have a top wall 36 and a bottom wall 38, as shown in Fig. 2. Referring to Fig. 7, top wall 36 and bottom wall 38 may be connected along seals or seams 34a, 34b, 34c and 34d to define the sealed compartments 30a, 30b and 30c (Fig. 2), which contain the vapor donating medium 32. The seals 34a, 34b, 34c and 34d may be sealed in any suitable manner, such as with adhesive or heating sealing.

Top and bottom walls 36 and 38 may both be made of a vapor permeable material. In other embodiments, one of top wall 36 and bottom wall 38 is made from a vapor permeable, liquid impermeable material while the other is made from a vapor and liquid impermeable material. The vapor and liquid impermeable material may be, for example, a multilayer laminated film and foil material (e.g., including a layer of aluminum foil). In yet other embodiments, as shown in Fig. 6, the sachets 28 may be tubes of vapor permeable, liquid impermeable material that are closed at each end.

When water is used as the hydrating medium, the amount of water contained within the sachets collectively is preferably an amount that can fully hydrate and maintain full hydration of the hydrophilic coating for at least the shelf life of the catheter. In some embodiments, each sachet may contain 0.5 mL/25 mm² of water or collectively the sachets together contain 8 mL of water. In one embodiment, the vapor donating medium is no more than about 20% of the volume of sealed cavity 15. Preferably, the amount of water contained within the sachets is sufficient to form and maintain a 100% relative humidity atmosphere within cavity 15. It is to be understood that the term "gas impermeable" in regard to the package is a relative term. Preferably, the package is enough of a barrier to moisture vapor to maintain a 100% relative humidity condition inside the package to ensure hydration of the hydrophilic coated catheter and maintenance of the hydration of the hydrated condition of the hydrophilic coated catheter for the desired shelf life of the packaged catheter assembly. The barrier properties required for this goal will depend on the length of the desired shelf life (typically between six months and five years), the amount of vapor donating medium or liquid placed in the package prior to sealing the package, and the conditions under which the product is stored.

In the package 12 illustrated in Figs. 1 and 2, there are three sachets 28a, 28b and 28c which are connected end-to-end and extend in a linear array. The sachets 28a - 28c may be connected by virtue of being made from the same piece or pieces of material, e.g., a sheet or layer of starting material forms a portion of each of the packets. Alternatively, the sachets may be connected in some other fashion, such as by adhesive or heat sealing the sachets together along the edges. The sachets may extend substantially along the entire length of the sealed cavity 15 of the package 12, which also may be, but not necessarily, extending substantially co-extensively with the length of catheter tube 14. Alternatively, the sachets 28a - 28c may extend substantially co-extensively with catheter tube 14 but not the length of the package. In other configurations, the package 12 may contain a plurality of separated individual sachets that are not connected to each other. In still other configurations, package 12 includes separate groups of sachets wherein the groups are separated but the sachets within the groups are connected to one another. Additionally, package 12 may contain at least two sachets or more than three sachets within cavity 12. In some embodiments, package 12 may contain substantially more sachets than the three sachets shown in Figs. 1 and 2. Also, the sachets 28a - 28c do not necessarily have to be connected end-to-end, but may be connected in other configurations as well.

Sachets 28a - 28c are illustrated as generally rectangular. The sachets, however, are not limited to rectangular and may be other regular and irregular geometric shapes. For example, the sachets may be round (as illustrated in Fig. 8), triangular or square. Furthermore, the sachets may have a variety of sizes. For example, each sachet may have a length and width between about 12 mm to about 50 mm. The sachet size also may be bigger or smaller and may even vary relative to other sachets placed within cavity 15.

The sachets are arranged within cavity 15 such that the vapor permeating from the sachets is substantially uniformly distributed at least along the length of catheter tube 14 containing the hydrophilic coating and preferably substantially uniform throughout the cavity 15 to thereby provide substantially uniform activation of the hydrophilic material of the medical device. Disposing the sachets at different locations along or throughout the package 12 achieves substantial uniform distribution of water vapor within the package 12 or at least along the length of the catheter tube 14. Optionally, the sachets are arranged or fixated relative to the cavity 15 of package 12 such that the sachets remain substantially stationary relative to the cavity 15 when the package 12 is moved or stored in different orientations. Such arrangement or fixation retains the vapor donating medium 32 at desired locations within the cavity 15 which assists in providing the substantially uniform distribution of water vapor regardless of the orientation of the package 12. For example, package 12 shown in Fig. 2 is in and may be stored in a substantially horizontal orientation, but the package 12 also may be orientated and/or stored in a substantially vertical orientation. When in the vertical orientation, sachets 28a, 28b and 28c retain the vapor donating water at substantially the same location within the package.

Referring to Figs. 1 and 2, the catheter assembly 10 optionally may include a thin, flexible, collapsible sleeve 22 preferably formed of a polymeric film that is vapor permeable (although it may be liquid impermeable) and through which the hydrophilic coating can be vapor hydrated. The sleeve 22 may be formed of any of a variety of thin, flexible polymeric film materials, such as polyethylene, plasticized PVC, or polypropylene, but elastomeric film materials such as polyurethane, and particularly elastomeric hydrogel materials, are believed particularly suitable. One such material is a polyurethane polyethylene oxide block copolymer commercially available under the trademark Medifilm® 435 from Mylan Labs, St. Albans, VT, but other elastomeric hydrogel films are known and may be used. Most desirably, the film is vapor permeable, since such vapor permeability promotes distribution of vapor within the package and facilitates vapor hydration of the catheter's hydrophilic coating. It is also preferred that the film be impermeable to liquid water, to ensure a complete barrier to microbe penetration, although a liquid permeable sleeve may in some instances be used.

The thickness of the film from which the sleeve is formed may vary considerably depending on factors such as stretchability and flexibility of the material selected but, in general, the thickness will fall within the range of about 10 to 150 microns, preferably about 13 to 50 microns. Similarly, the aging or incubating time required to achieve full vapor hydration depends on a number of variables such as the moisture vapor transmission rate (MTVR) of the material of the sleeve, the size of the package as a whole, the diameter of the sleeve in relation to other components such as the catheter tube, and the ambient temperatures and pressures involved. In any event, the time interval between packaging and use is both substantial (e.g., on the order of days or weeks, rather than seconds) and may be predetermined for any given product to ensure that the vapor donating medium within the package has vaporized sufficiently to produce a condition of 100% humidity-with complete vapor hydration of the hydrophilic coating-by the time the catheter is required for use.

During manufacture, the catheter assembly 10 and a desired number of water sachets are placed within cavity 15 of package 12. In the embodiments illustrated in Figs. 1 and 2, three sachets 28a - 28c are placed within cavity 15. In other embodiments two sachets or more than three sachets may be placed in cavity 15. Package 12 is then sealed. The presence of the vapor donating medium or liquid 32, such as water, within the sachets 28a - 28c causes vapor, such as water vapor, to be formed over a determinable period of time and permeate through the vapor permeable portion of the sachets. When flexible sleeve 22 is present, it is preferably made of a thin, flexible hydrogel material which has a high vapor transmission rate. Thus, the flexible hydrogel sleeve 22 permits the vapor created by the evaporating vapor donating medium located within the sachets to enter and hydrate or otherwise wet or activate the hydrophilic coating on the outer surface of the tube 14.

The hydrophilic coating on the outer surface of the tube 14 therefore becomes hydrated or activated by reason of exposure to the vapor. This activates the hydrophilic coating to create a highly lubricious condition on the outer surface of the tube 14 which places the assembly 10 in a ready-to-use condition. The assembly is aged for a predetermined period after completion of the packaging process, to ensure complete activation of the coating. The assembly can then be removed by the user from the package 12 and used immediately. Moreover, this can all be accomplished without the necessity for the user to add water and without the user encountering the prior art problems of water spillage when the package is opened.

Fig. 3 illustrates a package 12' of the present disclosure which includes pouches 28a', 28b' and 28c'. Package 12' includes a top wall 12a' and a bottom wall 12b' defining a sealed cavity 15'. In this embodiment, pouches 28a' - 28c' are formed from a top wall or layer 36' and bottom wall 12b' of the package 12'. Top wall 36' and at least a portion of bottom wall 12b' define the sealed compartments 30a', 30b' and 30c', which contain the vapor donating medium or liquid 32. Top wall or layer 36' is at least partially made from a vapor permeable, liquid impermeable material that allows vapor donated from the vapor donating medium 32 to permeate through the vapor permeable material and into cavity 15'. Similar to as described above, when optional flexible sleeve 22 is present, it permits the vapor created by the evaporating liquid located in the sachets 28a' - 28c' to enter and hydrate the hydrophilic coating on the outer surface of the tube 14. The hydrophilic coating on the outer surface of the tube 14 therefore becomes hydrated or activated by reason of exposure to the vapor. This activates the hydrophilic coating to create a highly lubricious condition on the outer surface of the tube 14 which places the catheter assembly 10 in a ready-to-use condition.

To use the catheter assembly 10, the user may simply remove it from the package 12 by gripping the sleeve 22, when present, and then gently insert the introducer tip 16, when present, into the urethral opening. Preferably, the catheter assembly 10 is gripped by the sleeve 22 in one hand for advancement of the formed tip 18 of the tube 14 into and through the introducer tip 16, such introducer tip having an opening 16a, such as a plurality of cross slits defining a circumferential array of flaps that flex outwardly to form the opening for allowing passage of the tube 14 therethrough. The opening 16a may have other configurations as well. Thereafter, the tube is gently advanced by using the other hand to grip the tube between wall portions of the sleeve and urge the tube forwardly or proximally. As the tube 14 advances through the urethral opening into the body, the sleeve 22 will crumple adjacent the funnel 24 of the catheter assembly 10.

Figs. 4 and 5 illustrate one method of manufacturing a plurality of connected sachets of the present disclosure which utilizes a vertical form fill and seal technique (VFFS). Referring first to Fig. 4, a first supply or roll 44 of a first material 46 and a second supply or roll 48 of a second material 50 are provided wherein at least one of the materials 46/50 is comprised of a vapor permeable, liquid impermeable material. When the other material is not a vapor permeable, liquid impermeable material, it may be any other suitable vapor and liquid impermeable material such as any of the above-described vapor and liquid impermeable materials. In one embodiment, one of the first and second materials 46/50 comprises the material that eventually forms a portion of the sachets 28a' - 26c' and the package 12' shown in Fig. 3.

Referring to Fig. 4, the first and second materials 46/50 are feed together and then a horizontal heat sealing element 52 seals the first and second materials 46/50 together to form a bottom seal 34a (Fig. 7) of a sachet 28. Referring to Fig. 5, after the bottom seal 34a is formed, the materials 46/50 are further feed together and a vertical heat sealer 54 seals the sides of the first and second material together to form seals 34c and 34d (Fig. 7). Turning back to Fig. 4, with the bottom and sides of the sachet sealed together, the vapor donating medium 32 is dispensed into the compartment defined by the sachet and then the horizontal heat sealer 52 creates a second horizontal or top seal 34b (Fig. 7) to seal the sachet having the liquid hydrating medium therein. The top seal 34b of a sachet may also serve as the bottom seal 34a of the next sachet formed. The process continues to form a string of connected sachets. The process may include cutting the string into groupings of a desired number of sachets immediately after formation. Alternatively, the process may include forming a long string of sachets and then cutting the sachets into desired groupings as needed.

In the embodiment wherein the sachets are discrete from the package, i.e., the package walls do not form a portion of the sachets (as shown in Fig. 2), the catheter package is assembled by cutting a desired number of sachets from the above-discussed string of sachets. The sachets may be cut into individual sachets or may be cut from the string as a group of connected sachets. The desired number of sachets is then placed in the catheter package along with the hydrophilic catheter, as described above.

In the embodiment wherein the one of the materials 46/50 forms the catheter package and part of the sachets, a group of sachets including the desired number of sachets or contained in a desired length of the package are cut from the string. The material that forms the catheter package is then folded or a second sheet of material is added over and sealed to the string to define the catheter package containing the sachets in cavity 15'as shown in Fig. 3. A hydrophilic catheter 14 is placed in the cavity and the package is sealed. The hydrophilic catheter 14 may be a urinary catheter for insertion into a human urethra, for example an intermittent catheter.

Fig. 6 illustrates another process for manufacturing sachets of the present disclosure that also uses a VFFS technique. This process uses only one roll 56 of material 58 wherein the material 58 is a vapor permeable, liquid impermeable material. The process includes joining the sides 60/62 of material 58 together to create a seal 64, which forms the material 58 into a tube-like structure. For example, the sides 60/62 of the material 58 may be heat sealed together. A horizontal heat sealer 66 is then used to form a bottom seal 68 of a sachet. Vapor donating medium 32 is then dispensed into the sachet and the horizontal heat sealer 66 forms a top seal 70 of the sachet to form the sealed sachet containing the hydrating material therein. Top seal 70 may be the bottom seal 68 of the next sachet to be formed. Similar to the process shown in Figs. 4 and 5, the process continues to form a string of a plurality of connected sachets which may be cut into separate sachets or groupings of connected sachets and placed in a catheter package along with a hydrophilic medical device.

It will be understood that the embodiments described above are illustrative of some of the applications of the principles of the present subject matter.

## Claims

1. A ready-to-use packaged vapor hydrated hydrophilic medical device assembly, comprising:
a package (12, 12') defining a sealed cavity (15, 15');
a medical device (14) comprising a hydrophilic material disposed within the cavity (15, 15'); and
**characterized in that**:
a plurality of individual sachets (28a-28c, 28a'-28c') disposed within the cavity (15, 15'), each of the sachets (28a-28c, 28a'-28c') including a top wall (36) and a bottom wall (38) that define a sealed compartment (30a-30c, 30a'-30c') wherein the compartment (30a-30c, 30a'-30c') of each sachet (28a-28c, 28a'-28c') is distinct from the compartments (30a-30c, 30a'-30c') of the other sachets (28a-28c, 28a'-28c'), at least one of the top and bottom walls (36, 38) being at least partially made of a vapor permeable, liquid impermeable material, and the sealed compartment (30a-30c, 30a'-30c') containing a vapor donating medium (32) therein, wherein vapor donated from the vapor donating medium (32) permeates through the vapor permeable material of the sachets (28a-28c, 28a'-28c') and into the sealed cavity (15, 15') defined by the package (12, 12') to activate the hydrophilic material of the medical device (14), wherein the plurality of sachets (28a-28c, 28a'-28c') extend along the length of the medical device within the cavity (15, 15') to provide substantially uniform activation of the hydrophilic material regardless of the orientation of the package (12, 12').

2. The medical device assembly of claim 1, wherein the sachets (28a-28c, 28a'-28c') are connected to each other.

3. The medical device assembly of any one of claims 1 and 2 wherein the sachets (28a-28c, 28a'-28c') are arranged in a substantially linear array.

4. The medical device assembly of any one of the preceding claims wherein the sachets (28a-28c, 28a'-28c') have a generally rectangular, triangular or arcuate shape.

5. The medical device assembly of any one of the preceding claims wherein the vapor donating medium (32) comprises liquid water.

6. The medical device assembly of any one of the preceding claims wherein the medical device (14) comprises an intermittent urinary catheter.

7. The medical device assembly of any one of the preceding claims wherein each sachet (28a-28c, 28a'-28c') contains about 0.5 mL/25mm² of vapor donating medium (32).

8. The medical device assembly of any one of the preceding claims wherein the package (12, 12') is comprised of a gas impermeable material.

9. A method of manufacturing a packaged vapor hydrated hydrophilic medical device assembly, comprising:
placing a medical device (14) comprising a hydrophilic material into a cavity (15, 15') of a package (12, 12');
**characterized in that**:
placing a plurality of individual sachets (28a-28c, 28a'-28c') containing a vapor donating medium (32) within the cavity of the package (12, 12'), wherein each of the sachets (28a-28c, 28a'-28c') includes a top wall (36) and a bottom wall (38) that define a sealed compartment (30a-30c, 30a'-30c') wherein the compartment (30a-30c, 30a'-30c') of each sachet (28a-28c, 28a'-28c') is distinct from the compartments (30a-30c, 30a'-30c') of the other sachets (28a-28c, 28a'-28c'), at least one of the top and bottom walls (36, 38) being at least partially formed of a vapor permeable, liquid impermeable material; and
sealing the package (12, 12'), wherein the plurality of sachets (28a-28c, 28a'-28c') is arranged within the cavity (15, 15') to extend along the length of the medical device to provide substantially uniform activation of the hydrophilic material of the medical device regardless of the orientation of the package.

10. The method of claim 9, wherein the sachets (28a-28c, 28a'-28c') are connected to each other.

11. The method of any one of claims 9 and 10, wherein the sachets (28a-28c, 28a'-28c') are placed within the package (12, 12') in a substantially linear array.

12. The method of any one of claims 9-11, wherein the medical device (14) comprises an intermittent urinary catheter.

13. The method of any one of claims 9-12, wherein each sachet (28a-28c, 28a'-28c') contains about 0.5 mL/25mm² of vapor donating medium (32).

## Patentansprüche

1. Gebrauchsfertige, verpackte, dampfhydratisierte, hydrophile Medizinproduktbaugruppe, umfassend:
eine Verpackung (12, 12'), die einen abgedichteten Hohlraum (15, 15') definiert;
ein Medizinprodukt (14), das ein hydrophiles Material umfasst, das in dem Hohlraum (15, 15') angeordnet ist; und
**dadurch gekennzeichnet, dass**:
eine Vielzahl von einzelnen Beuteln (28a-28c, 28a'-28c') innerhalb des Hohlraums (15, 15') angeordnet ist, wobei jeder der Beutel (28a-28c, 28a'-28c') eine obere Wand (36) und eine untere Wand (38) beinhaltet, die eine abgedichtete Kammer (30a-30c, 30a'-30c') definieren, wobei sich die Kammer (30a-30c, 30a'-30c') eines jeden Beutels (28a-28c, 28a'-28c') von den Kammern (30a-30c, 30a'-30c') der anderen Beutel (28a-28c, 28a'-28c') unterscheidet, wobei mindestens eine von der oberen und der unteren Wand (36, 38) mindestens teilweise aus einem dampfdurchlässigen, flüssigkeitsundurchlässigen Material besteht, und die abgedichtete Kammer (30a-30c, 30a'-30c') ein Dampfabgabemedium (32) enthält, wobei Dampf, der von dem Dampfabgabemedium (32) abgegeben wird, durch das dampfdurchlässige Material der Beutel (28a-28c, 28a'-28c') und in den abgedichteten Hohlraum (15, 15') dringt, der durch die Verpackung (12, 12') definiert ist, um das hydrophile Material des Medizinprodukts (14) zu aktivieren, wobei sich die Vielzahl von Beuteln (28a-28c, 28a'-28c') entlang der Länge des Medizinprodukts innerhalb des Hohlraums (15, 15') erstreckt, um eine im Wesentlichen gleichmäßige Aktivierung des hydrophilen Materials unabhängig von der Ausrichtung der Verpackung (12, 12') bereitzustellen.

2. Medizinproduktbaugruppe nach Anspruch 1, wobei die Beutel (28a-28c, 28a'-28c') miteinander verbunden sind.

3. Medizinproduktbaugruppe nach einem der Ansprüche 1 und 2, wobei die Beutel (28a-28c, 28a'-28c') in einer im Wesentlichen linearen Anordnung angeordnet sind.

4. Medizinproduktbaugruppe nach einem der vorhergehenden Ansprüche, wobei die Beutel (28a-28c, 28a'-28c') eine im Allgemeinen rechteckige, dreieckige oder bogenförmige Gestalt aufweisen.

5. Medizinproduktbaugruppe nach einem der vorhergehenden Ansprüche, wobei das Dampfabgabemedium (32) flüssiges Wasser umfasst.

6. Medizinproduktbaugruppe nach einem der vorhergehenden Ansprüche, wobei das Medizinprodukt (14) einen intermittierenden Harnkatheter umfasst.

7. Medizinproduktbaugruppe nach einem der vorhergehenden Ansprüche, wobei jeder Beutel (28a-28c, 28a'-28c') etwa 0,5 ml/25 mm² Dampfabgabemedium (32) enthält.

8. Medizinproduktbaugruppe nach einem der vorhergehenden Ansprüche, wobei die Verpackung (12, 12') aus einem gasundurchlässigen Material besteht.

9. Verfahren zum Herstellen einer verpackten, dampfhydratisierten, hydrophilen Medizinproduktbaugruppe, umfassend:
Platzieren eines Medizinprodukts (14), das ein hydrophiles Material umfasst, in einem Hohlraum (15, 15') einer Verpackung (12, 12');
**gekennzeichnet durch**:
Platzieren einer Vielzahl von einzelnen Beuteln (28a-28c, 28a'-28c'), die ein Dampfabgabemedium (32) enthalten, innerhalb des Hohlraums der Verpackung (12, 12'), wobei jeder der Beutel (28a-28c, 28a'-28c') eine obere Wand (36) und eine untere Wand (38) beinhaltet, die eine abgedichtete Kammer (30a-30c, 30a'-30c') definieren, wobei sich die Kammer (30a-30c, 30a'-30c') eines jeden Beutels (28a-28c, 28a'-28c') von den Kammern (30a-30c, 30a'-30c') der anderen Beutel (28a-28c, 28a'-28c') unterscheidet, wobei mindestens eine von der oberen und der unteren Wand (36, 38) mindestens teilweise aus einem dampfdurchlässigen, flüssigkeitsundurchlässigen Material besteht; und
Abdichten der Verpackung (12, 12'), wobei die Vielzahl von Beuteln (28a-28c, 28a'-28c') innerhalb des Hohlraums (15, 15') derart angeordnet ist, dass sie sich entlang der Länge des Medizinprodukts erstreckt, um eine im Wesentlichen gleichmäßige Aktivierung des hydrophilen Materials des Medizinprodukts unabhängig von der Ausrichtung der Verpackung bereitzustellen.

10. Verfahren nach Anspruch 9, wobei die Beutel (28a-28c, 28a'-28c') miteinander verbunden sind.

11. Verfahren nach einem der Ansprüche 9 und 10, wobei die Beutel (28a-28c, 28a'-28c') in der Verpackung (12, 12') in einer im Wesentlichen linearen Anordnung platziert werden.

12. Verfahren nach einem der Ansprüche 9-11, wobei das Medizinprodukt (14) einen intermittierenden Harnkatheter umfasst.

13. Verfahren nach einem der Ansprüche 9-12, wobei jeder Beutel (28a-28c, 28a'-28c') etwa 0,5 ml/25 mm² Dampfabgabemedium (32) enthält.

## Revendications

1. Ensemble dispositif médical hydrophile à hydratation par vapeur emballé prêt à l'emploi, comprenant :
un emballage (12, 12') définissant une cavité scellée (15, 15');
un dispositif médical (14) comprenant un matériau hydrophile disposé à l'intérieur de la cavité (15, 15') ; et
**caractérisé en ce que** :
une pluralité de sachets individuels (28a-28c, 28a'-28c') sont disposés à l'intérieur de la cavité (15, 15'), chacun des sachets (28a-28c, 28a'-28c') comprenant une paroi supérieure (36) et une paroi inférieure (38) qui définissent un compartiment scellé (30a-30c, 30a'-30c') dans lequel le compartiment (30a-30c, 30a'-30c') de chaque sachet (28a-28c, 28a'-28c') est distinct des compartiments (30a-30c, 30a'-30c') des autres sachets (28a-28c, 28a'-28c'), au moins l'une des parois supérieure et inférieure (36, 38) étant constituée au moins partiellement d'un matériau imperméable aux liquides et perméable à la vapeur, et le compartiment scellé (30a-30c, 30a'-30c') contenant un milieu produisant de la vapeur (32) à l'intérieur, dans lequel la vapeur produite par le milieu produisant de la vapeur (32) pénètre à travers le matériau perméable à la vapeur des sachets (28a-28c, 28a'-28c') et dans la cavité scellée (15, 15') définie par l'emballage (12, 12') pour activer le matériau hydrophile du dispositif médical (14), dans lequel la pluralité de sachets (28a-28c, 28a'-28c') s'étendent le long du dispositif médical à l'intérieur de la cavité (15, 15') pour permettre une activation sensiblement uniforme du matériau hydrophile quelle que soit l'orientation de l'emballage (12, 12').

2. Ensemble dispositif médical selon la revendication 1, dans lequel les sachets (28a-28c, 28a'-28c') sont reliés les uns aux autres.

3. Ensemble dispositif médical selon l'une quelconque des revendications 1 et 2, dans lequel les sachets (28a-28c, 28a'-28c') sont disposés dans un réseau sensiblement linéaire.

4. Ensemble dispositif médical selon l'une quelconque des revendications précédentes, dans lequel les sachets (28a-28c, 28a'-28c') ont une forme généralement rectangulaire, triangulaire ou arquée.

5. Ensemble dispositif médical selon l'une quelconque des revendications précédentes, dans lequel le milieu produisant de la vapeur (32) comprend de l'eau liquide.

6. Ensemble dispositif médical selon l'une quelconque des revendications précédentes, dans lequel le dispositif médical (14) comprend un cathéter urinaire intermittent.

7. Ensemble dispositif médical selon l'une quelconque des revendications précédentes, dans lequel chaque sachet (28a-28c, 28a'-28c') contient environ 0,5 ml/25 mm² de milieu produisant de la vapeur (32).

8. Ensemble dispositif médical selon l'une quelconque des revendications précédentes, dans lequel l'emballage (12, 12') est constitué d'un matériau imperméable aux gaz.

9. Procédé de fabrication d'un ensemble dispositif médical hydrophile à hydratation par vapeur emballé, comprenant :
le placement d'un dispositif médical (14) comprenant un matériau hydrophile dans une cavité (15, 15') d'un emballage (12, 12') ;
**caractérisé par** :
le placement d'une pluralité de sachets individuels (28a-28c, 28a'-28c') contenant un milieu produisant de la vapeur (32) à l'intérieur de la cavité de l'emballage (12, 12'), dans lequel chacun des sachets (28a-28c, 28a'-28c') comprend une paroi supérieure (36) et une paroi inférieure (38) qui définissent un compartiment scellé (30a-30c, 30a'-30c') dans lequel le compartiment (30a-30c, 30a'-30c') de chaque sachet (28a-28c, 28a'-28c') est distinct des compartiments (30a-30c, 30a'-30c') des autres sachets (28a-28c, 28a'-28c'), au moins l'une des parois supérieure et inférieure (36, 38) étant au moins partiellement formée d'un matériau imperméable aux liquides et perméable à la vapeur ; et
le scellement de l'emballage (12, 12'), dans lequel la pluralité de sachets (28a-28c, 28a'- 28c') sont agencés à l'intérieur de la cavité (15, 15') pour s'étendre le long du dispositif médical afin de permettre une activation sensiblement uniforme du matériau hydrophile du dispositif médical quelle que soit l'orientation de l'emballage.

10. Procédé selon la revendication 9, dans lequel les sachets (28a-28c, 28a'-28c') sont reliés les uns aux autres.

11. Procédé selon l'une quelconque des revendications 9 et 10, dans lequel les sachets (28a-28c, 28a'-28c') sont placés à l'intérieur de l'emballage (12, 12') dans un réseau sensiblement linéaire.

12. Procédé selon l'une quelconque des revendications 9 à 11, dans lequel le dispositif médical (14) comprend un cathéter urinaire intermittent.

13. Procédé selon l'une quelconque des revendications 9 à 12, dans lequel chaque sachet (28a-28c, 28a'- 28c') contient environ 0,5 ml/25 mm² de milieu produisant de la vapeur (32).
